# FASCICULE DE BREVET EUROPEEN

(11) **EP 1 572 148 B1**
(45) Date de publication et mention de la délivrance du brevet: **18.11.2009**
(21) Numéro de dépôt: 03815111.4
(22) Date de dépôt: 10.12.2003
(51) Int. Cl.: A61K 8/64, A61K 8/97, A61Q 19/08

(54) **COMPOSITION COSMETIQUE COMPRENANT UN EXTRAIT DE SIEGESBECKIA ET UN LIPOPEPTIDE**
KOSMETISCHE ZUSAMMENSETZUNG ENTHALTEND EIN SIEGESBECKIA EXTRAKT UND EIN LIPOPEPTID
COSMETIC COMPOSITION COMPRISING A SIEGESBECKIA EXTRACT AND A LIPOPEPTIDE

(30) Priorité: 10.12.2002 FR 0215582
(43) Date de publication de la demande: 14.09.2005
(73) Titulaire: Laboratoire Nuxe, 75010 Paris (FR)
(72) Inventeur: LECLERE, Jacques, F-45500 SAINT-GONDON (FR)
(74) Mandataire: L'Helgoualch, Jean
(86) Numéro de dépôt international: PCT/FR2003/003660
(87) Numéro de publication internationale: WO 2004/062637

(56) Documents cités:
- FR-A- 2 285 142
- FR-A- 2 783 169
- FR-A- 2 813 018

## Description

La présente invention concerne une nouvelle composition cosmétique et/ou dermatologique, et plus particulièrement une composition à base d'un inhibiteur des métallo-protéinases matricielles (MMP) constitué par un extrait de Siegesbeckia, et d'un lipopeptide constitué par la palmitoyl-lysyl-thréonyl-thréonyl-lysyl-serine, présentant d'excellentes propriétés utilisables en cosmétique et en dermatologie pour les soins et la protection de la peau contre les signes de vieillissement.

La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. Le derme sert de support à l'épiderme et est principalement constitué de fibroblastes et d'une matrice extracellulaire essentiellement à base collagène et d'élastine. Les fibres de collagène contribuent à la tonicité et l'élasticité de la peau. Avec l'âge, leur renouvellement diminue, ce qui se traduit par un amincissement de la peau, et leur perte progressive d'élasticité entraîne un durcissement de la peau. L'épiderme, qui est composé de trois types de cellules dont les plus importantes sont les kératinocytes, constitue la couche externe, et joue un rôle fondamental pour assurer la protection et le maintien d'une bonne trophicité. C'est pourquoi de nombreuses compositions ont été mises au point afin de le protéger et d'améliorer les fonctions de la peau.

On sait que le vieillissement de la peau s'accompagne d'un certain nombre de signes et en particulier de la formation de rides plus ou moins profondes et étendues, outre une perte d'élasticité et un amincissement. L'apparition des premières rides, qui peut survenir chez des individus dès l'âge de 30 à 40 ans, est un phénomène qui peut être aggravé par des agressions physiques ou chimiques provenant de la pollution, de l'exposition aux rayons ultraviolets ou des modes de vie qui accélèrent le vieillissement cutané.

Dans les tissus normaux, existe un équilibre entre dégradation et synthèse tissulaire. Ainsi la matrice extracellulaire peut être dégradée par des métallo-protéinases qui se répartissent essentiellement en trois groupes principaux qui sont les collagénases, les gélatinases et stromelysines. Par exemple, la fibroblaste collagénase (MMP-1) fait partie des collagénases, la gélatinase A (MMP-2) et la gélatinase B (MMP-9) sont des gélatinases, et la stromelysine-1 (MMP-3) et la matrilysine (MMP-7) sont des stromelysines. Un excès de métallo-protéinases entraîne une dégradation de biomolécules telles que collagène, protéoglycanne et gélatine, qui peut avoir des conséquences néfastes sur le tissu épidermique et peut aussi engendrer des maladies des cartilages, des processus inflammatoires, des mélanomes, etc. Aussi, de nombreuses études ont été consacrées aux propriétés inhibitrices des métallo-protéinases de diverses substances.

D'une manière générale, le traitement et la prévention des signes du vieillissement cutané sont l'objet de nombreux travaux et études depuis des années, notamment pour mettre au point des compositions susceptibles de favoriser la restructuration tissulaire, et en particulier la néosynthèse d'éléments constitutifs de la peau comme le collagène.

Par ailleurs, on sait que, pour être acceptées par les utilisateurs, les compositions cosmétiques et/ou dermatologiques destinées au traitement et à la prévention des affections de la peau par application topique doivent être agréables à utiliser et présenter de bonnes propriétés physiques, notamment de consistance et d'onctuosité, tout en garantissant une efficacité satisfaisante et en évitant ou masquant les inconvénients tels que tiraillements, irritations ou démangeaisons, que peuvent occasionner certains principes actifs.

Dans l'état de la technique, par exemple le brevet FR-A-2.761.607 décrit une composition dermatologique destinée au traitement des symptômes du vieillissement de la peau, comportant un dérivé de silanol méthylé et un dérivé d'une protéine végétale hydrolysée, additionnée le cas échéant d'un dérivé de vitamine C. Le brevet EP-A-662.319 décrit des compositions cosmétiques et dermatologiques comprenant un céramide comme agent apaisant pour compenser l'effet irritant du principe actif anti-vieillissement. Le brevet FR-A-2.783.169 décrit l'utilisation de pentapeptides du type Lys-Thr-Thr-Lys-Ser dans des compositions topiques pour favoriser la synthèse du collagène et des glycosaminoglycannes, et par conséquent la régénération cutanée. Un pentapeptide correspondant à cette formule est disponible dans le commerce sous la marque Matrixyl. Le brevet FR-A-2.813.018 décrit une composition contenant de l'acide ursolique, ou son isomère, l'acide oléanique, c'est-à-dire un triterpène dérivé du romarin, que l'on peut associer au Matrixyl, et éventuellement à un extrait peptidique de lupin blanc, pour renforcer la jonction dermoépidermique.

L'invention a pour objet une composition cosmétique et/ou dermatologique à base d'extrait de Siegesbeckia et de lipopeptide constitué par la palmitoyl-lysyl-thréonyl-thréonyl-lysyl-sérine.

Enfin, la présente invention a encore pour objet un procédé de traitement cosmétique et cosmétologique des signes du vieillissement de la peau consistant à appliquer sur la peau une composition comme indiqué ci-dessus.

Les études effectuées par la demanderesse ont montré de manière inattendue que l'association d'un inhibiteur des métallo-protéinases tel qu'un extrait de Siegesbeckia avec le lipopeptide constitué par la palmitoyl-lysyl-thréonyl-thréonyl-lysyl-sérine avait pour effet de potentialiser l'activité de ce dernier sur la néosynthèse de collagène.

Chacun des deux composants essentiels indiqués ci-dessus possède sa propre activité, mais on a constaté que leur combinaison au sein d'une même composition procurait des effets nettement supérieurs à ceux que l'on peut obtenir en utilisant les composants séparément.

L'inhibiteur des métallo-protéinases (MMP), ou anti-métallo-protéinase, utilisé dans les compositions de la présente invention est constitué par un extrait de Siegesbeckia, et plus préférentiellement de Siegesbeckia orientalis. Suivant l'invention, on utilise un inhibiteur des métallo-protéinases choisi parmi les inhibiteurs actifs sur les MMP-1 et/ou les MMP-3. La composition de l'invention ne contient pas d'extrait peptidique de lupin blanc bien que ce dernier ait été décrit comme possédant des propriétés inhibitrices des métallo-protéinases.

Le lipopeptide utilisé dans les compositions suivant la présente invention comporte la séquence palmitoyl-Lys-Thr-Thr-Lys-Ser, et on peut choisir par exemple le lipopeptide disponible dans le commerce sous la marque Matrixyl (Sederma) généralement sous forme de solution hydroalcoolique titrée en palmitoyl-lysyl-thréonyl-thréonyl-lysyl-sérine. Un tel lipopeptide est décrit dans le brevet FR-A-2.783.169 précité.

L'extrait de Siegesbeckia, et en particulier Siegesbeckia orientalis, peut être obtenu suivant la méthode décrite dans le brevet FR-A-2.285.142. Ses propriétés sont essentiellement dues à la présence d'un dérivé diterpénique dénommé darutoside qui est un glucoside de darutigénol, et exerce une activité sur les métalloprotéinases, permettant de l'utiliser pour lutter contre les processus inflammatoires de la peau et protéger le collagène contre une dégradation trop rapide initiée par les radicaux libres.

La combinaison de lipopeptide et d'inhibiteur de métallo-protéinase, en particulier d'extrait de Siegesbeckia, selon la présente invention favorise fortement la néosynthèse de collagène en raison de l'expression de métallo protéinases de type 1 (MMP-1) et/ou de type 3 (MMP-3). Les études effectuées par la demanderesse ont montré que la synthèse de collagène, en particulier de collagène de type I, IV et VII, était augmentée de 28% par rapport à l'utilisation de lipopeptide isolément.

De plus, on constate qu'une composition ayant une teneur en poids de 0,1% en combinaison Matrixyl / extrait de Siegesbeckia orientalis (1:1) diminue de 23% la production de métallo protéinases de type 1.

La combinaison de lipopeptide et d'inhibiteur de métallo-protéinase, en particulier d'extrait de Siegesbeckia, selon la présente invention a pour effet de favoriser la néosynthèse de collagène, comme indiqué ci-dessus, et peut être incorporée dans des compositions cosmétiques et dermatologiques pour renforcer leur pouvoir antirides, augmenter l'effet d'hydratation de la peau et améliorer la souplesse et l'élasticité des tissus de la peau.

Les quantités utilisées dans les compositions cosmétiques sont généralement de l'ordre de 0,1 à 15% environ, et de préférence de 3 à 10% en poids, pour le lipopeptide, et de 0,1 à 10%, de préférence de 2 à 6% en poids, pour l'inhibiteur des métallo-protéinases, en particulier l'extrait de Siegesbeckia.

Le lipopeptide et l'extrait de Siegesbeckia peuvent être utilisés en quantités sensiblement équivalentes, le rapport en poids lipopeptide / extrait de Siegesbeckia étant généralement compris entre 5:1 et 1:2.

Il peut être avantageux, suivant une variante de la présente invention, d'incorporer dans la composition une substance présentant des propriétés utiles en complément, par exemple une activité anti-élastase, telle que l'acide oléique ou un extrait de Boswellia serrata.

Les compositions suivant la présente invention sont administrables par voie topique et elles peuvent avantageusement contenir, outre les composants de base décrits ci-dessus, une ou plusieurs autres substances connues pour exercer des effets complémentaires bénéfiques pour la peau, et par exemple le tocophérol, la vitamine A (rétinol), l'acide rétinoïque, des agents bactéricides, etc.

Les compositions cosmétiques ou dermatologiques conformes à la présente invention contiennent des supports et excipients couramment utilisés dans des compositions destinées à l'administration topique, telles que des émulsions H/E ou E/H, des crèmes, des gels ou des lotions. Dans le cas des émulsions, la phase grasse peut représenter entre 10 et 60% environ du poids de la composition, la phase aqueuse entre 10 et 80% environ et l'agent émulsionnant entre 2 et 20%, le reste étant constitué par les composants de base indiqués ci-dessus et les autres composants mentionnés ci-après.

Les compositions peuvent encore contenir diverses substances et excipients choisis en fonction de leurs propriétés connues et de la forme galénique envisagée. Ainsi, on peut incorporer des agents hydratants, des agents émulsionnants, des tensioactifs, des épaississants, des gélifiants, des agents viscosants, des conservateurs, des antioxydants, des parfums des huiles, des lipides, un solvant spécifique ainsi que de l'eau et divers additifs destinés à améliorer les propriétés physiques des compositions. On peut encore avantageusement incorporer des filtres ou écrans solaires choisis en fonction du degré de protection recherché.

L'agent hydratant peut être choisi parmi les produits connus dans la préparation de compositions utilisables en cosmétologie et en dermatologie, et par exemple on peut utiliser un polyol, la glycérine (glycérol et des dérivés de glycérol), des alkylène polyols tels que le polyéthylène glycol, le sorbitol, le maltitol, le penta-érythritol, les polyacrylates et polyméthacrylates de glycéryle, les mucopolysaccharides tels que l'acide hyaluronique, des dérivés du chitosan et des dérivés de l'acide pyrrolidone carboxylique. L'agent hydratant est avantageusement introduit dans la phase aqueuse lors de la préparation de l'émulsion. La teneur en agent hydratant est généralement comprise entre 0,1 et 10% en poids par rapport au poids total de l'émulsion.

Les compositions suivant la présente invention peuvent être présentées sous les formes classiquement utilisées pour une application topique, c'est-à-dire sous forme de gel, lotion, émulsion (en particulier crème ou lait), masque, stick ou pommade, contenant les composants décrits ci-dessus, et des excipients et supports usuels compatibles et pharmaceutiquement acceptables. Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

Les exemples suivants illustrent plus en détail l'invention sans en limiter la portée. Dans tous les exemples de compositions qui suivent, les parties sont exprimées en poids, sauf indication contraire.

### Exemple 1

On prépare un gel anti-âge ayant la composition suivante.

| | | |
|---|---|---|
| Matrixyl® | | 5,00 |
| Extrait de Siegesbeckia orientalis | | 3,00 |
| Protéines de soja hydrolysées | | 15,00 |
| Acide hyaluronique | | 0,10 |
| Butylène glycol-1,3 | | 4,50 |
| Glycérine | | 5,00 |
| Carbomer | | 0,20 |
| Trisamino | | 0,22 |
| Phénoxyéthanol | | 0,50 |
| PEG-60 huile de ricin hydrogénée | | 0,25 |
| Dipropylène glycol | | 2,00 |
| Conservateurs | | 0,25 |
| Parfums | | 0,10 |
| Eau | q.s.p. | 100,00 |

Ce gel peut être appliqué deux ou trois fois par jour sur le visage et sur le corps.

### Exemple 2

Suivant les techniques classiques, on prépare une crème antirides ayant la composition pondérale suivante.

| | | |
|---|---|---|
| Matrixyl® | | 8,00 |
| Extrait de Siegesbeckia orientalis | | 3,00 |
| Extrait de Centaurum erythraea | | 2,00 |
| Stéaryl glucoside | | 3,00 |
| PGE-20 stéaryl glucoside | | 2,50 |
| Alcool béhénylique | | 2,50 |
| Phénoxyéthanol | | 0,50 |
| Protéines de soja hydrolysées | | 15,00 |
| Huile de maïs | | 3,00 |
| Isononanoate d'isononyle | | 2,00 |
| Huile de rosier muscat | | 1,00 |
| Beurre de mangue | | 1,00 |
| Gomme xanthane | | 0,50 |
| Butylène glycol-1,3 | | 5,00 |
| Acide hyaluronique | | 0,10 |
| Conservateur | | 0,30 |
| Parfums | | 0,10 |
| Eau | q.s.p. | 100,00 |

Cette composition est appliquée sur la peau en plusieurs applications quotidiennes.

### Exemple 3

Suivant les techniques classiques, on prépare une crème fluide antirides ayant la composition pondérale suivante.

| | | |
|---|---|---|
| Matrixyl® | | 5,00 |
| Extrait de Siegesbeckia orientalis | | 3,00 |
| PEG-20 monostéarate de sorbitane | | 3,50 |
| Monostéarate de sorbitane | | 2,50 |
| Perhydro squalène | | 5,00 |
| Protéines de soja hydrolysées | | 10,00 |
| Protéines de pois | | 5,00 |
| Palmitate d'isopropyle | | 2,00 |
| Monostéarate de diéthylène glycol | | 2,00 |
| Alcool cétéarylique | | 0,30 |
| Phénoxyéthanol | | 0,50 |
| Carbomer | | 0,15 |
| Trisamino | | 0,20 |
| Extrait de miel | | 3,00 |
| Conservateur | | 0,30 |
| Parfums | | 0,10 |
| Eau | q.s.p. | 100,00 |

Cette composition peut être appliquée sur la peau en plusieurs applications quotidiennes.

## Revendications

1. Composition cosmétique et/ou dermatologique **caractérisée en ce qu'**elle comprend un extrait de Siegesbeckia et un lipopeptide constitué par la palmitoyl-lysyl-thréonyl-thréonyl-lysyl-sérine.

2. Composition selon la revendication 1, **caractérisée en ce que** l'extrait de Siegesbeckia est un extrait de Siegesbeckia orientalis.

3. Composition selon l'une quelconque des revendications précédentes, **caractérisée en ce qu'**elle comprend de 0,1 à 15% en poids de lipopeptide, et de 0,1 à 10% en poids d'extrait de Siegesbeckia.

4. Composition selon la revendication 3, **caractérisée en ce que** le rapport lipopeptide / extrait de Siegesbeckia est compris entre 5:1 et 1:2.

5. Composition selon l'une quelconque des revendications précédentes, **caractérisée en ce qu'**elle comprend en outre une substance à activité anti-élastase choisie parmi l'acide oléique et un extrait de Boswellia serrata.

6. Procédé de traitement cosmétique des signes du vieillissement de la peau, **caractérisé en ce qu'**il consiste à appliquer sur la peau une composition selon l'une quelconque des revendications 1 à 5.

## Claims

1. A cosmetic and/or dermatological composition **characterized in that** it comprises an extract of *Siegesbeckia* and a lipopeptide formed by palmitoyl-lysyl-threonyl-threonyl-lysyl-serine.

2. The composition according to claim 1, **characterized in that** the extract of *Siegesbeckia* is an extract of *Siegesbeckia orientalis.*

3. The composition according to any of the preceding claims, **characterized in that** it comprises from 0.1% to 15% by weight of the lipopeptide, and from 0.1 to 15% by weight of *Siegesbeckia* extract.

4. The composition according to claim 3, **characterized in that** the lipopeptide/*Siegesbeckia* extract ratio is comprised between 5:1 and 1:2.

5. The composition according to any of the preceding claims, **characterized in that** it further comprises a substance with anti-elastase activity selected from oleic acid and an extract of *Boswellia serrata.*

6. A method for cosmetically treating signs of skin ageing, **characterized in that** it consists of applying on the skin a composition according to any of claims 1 to 5.

## Patentansprüche

1. Kosmetische und/oder dermatologische Zusammensetzung, **dadurch gekennzeichnet, dass** sie einen Siegesbeckia-Extrakt und ein aus Palmitoyl-Lysyl-Threonyl-Threonyl-Lysyl-Serin bestehendes Lipopeptid umfasst.

2. Zusammensetzung nach Anspruch 1, **dadurch gekennzeichnet, dass** der Siegesbeckia-Extrakt ein Extrakt von Siegesbeckia orientalis ist.

3. Zusammensetzung nach einem der vorangegangenen Ansprüche, **dadurch gekennzeichnet, dass** sie 0,1 bis 15 Gew.-% Lipopeptid und 0,1 bis 10 Gew.-% Siegesbeckia-Extrakt umfasst.

4. Zusammensetzung nach Anspruch 3, **dadurch gekennzeichnet, dass** das Verhältnis Lipopeptid:Siegesbeckia-Extrakt zwischen 5:1 und 1:2 liegt.

5. Zusammensetzung nach einem der vorangegangenen Ansprüche, **dadurch gekennzeichnet, dass** sie weiters eine Substanz mit Anti-Elastase-Aktivität umfasst, die aus Ölsäure und einem Extrakt von Boswellia serrata ausgewählt ist.

6. Verfahren zur kosmetischen Behandlung von Anzeichen der Hautalterung, **dadurch gekennzeichnet, dass** es darin besteht, eine Zusammensetzung nach einem der Ansprüche 1 bis 5 auf die Haut aufzutragen.
